# EUROPEAN PATENT SPECIFICATION

(11) **EP 1 885 688 B1**
(45) Date of publication and mention of the grant of the patent: **22.09.2010**
(21) Application number: 06770147.4
(22) Date of filing: 09.05.2006
(51) Int. Cl.: C07C 231/18

(54) **ASYMMETRIC HYDROGENATION FOR THE PREPARATION OF DIPHENYLALANINE DERIVATIVES**
ASYMMETRISCHE HYDRIERUNG ZUR HERSTELLUNG VON DIPHENYLALANINDERIVATEN
HYDROGENATION ASYMETRIQUE PERMETTANT LA PREPARATION DE DERIVES DE DIPHENYLALANINE

(30) Priority: 20.05.2005 US 683150 P
(43) Date of publication of application: 13.02.2008
(73) Proprietor: Dr. Reddy's Laboratories (EU) Limited, Beverley East Yorkshire HU17 0LD (GB)
(72) Inventor: BOULTON, Lee, T., Cambridge CB1 3JG (GB)
(74) Representative: Raynor, John
(86) International application number: PCT/US2006/017978
(87) International publication number: WO 2006/127273

(56) References cited:
- US-A- 5 198 548
- P. J. PYE, K. ROSSEN, R. A. REAMER, N. N. TSOU, R. P. VOLANTE AND P. J. REIDER: "A new planar chiral bisphosphine ligand for asymmetric catalysis: highly enantioselective hydrogenations under mild conditions" JOURNAL OF THE AMERICAN CHEMICAL SOCIETY, vol. 119, no. 26, 1997, pages 6207-6208, XP002401455 cited in the application

## Description

### BACKGROUND OF THE INVENTION

### Field of the Invention

The invention relates to an asymmetric hydrogenation process and, more particularly, to the use of a chiral-diphosphine rhodium homogeneous hydrogenation catalyst to achieve improved catalyst loadings and shorter reaction times in such an asymmetric hydrogenation process.

### Description of the Prior Art

Unnatural α-amino acids form an important class of building blocks for the synthesis of active pharmaceutical ingredients (APIs). For example, the metabolic stability of peptide and peptide-like APIs, and hence bioavailability, may be improved by incorporating one or more such amino acids. Unnatural α-amino acids can be further categorized into several structural sub-types including 3,3-diarylalanines **(I),** in which the two aryl substituents are typically the same. Several applications of these compounds have been reported. For example, a 3,3-diphenylalanine residue has been incorporated into thrombin inhibitors (Cody et al., Bioorg. Med. Chem. Lett., 1999, 9, 2497-2502), factor IIa antagonists (WO 2004073747) and HIV aspartyl protease inhibitors (WO 2004056764).

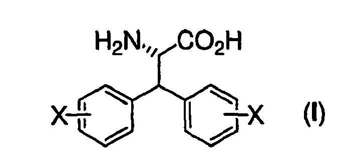

The use of 3,3-diarylalanines in novel APIs drives the requirement for efficient, scaleable and economic synthetic routes that provide single enantiomer forms. Existing routes reported in the literature have various disadvantages. The route to (*S*)-diphenylalanine reported by Josien et al (Tetrahedron Lett., 1991, 32, 6547-6550) requires use of a stoichiometric chiral auxiliary and a cryogenic alkylation step. Classical resolution approaches to 3,3-diphenylalanine and substituted analogues reported by Beylin et al. (US 5198548) are inherently inefficient, requiring disposal or reprocessing of the unwanted enantiomer, a shortcoming exacerbated at manufacturing scale. Accordingly, there is a need for more effective ways to obtain (*S*)- or (*R*)-3,3-diarylalanines and derivatives bearing suitable protecting and/or activating groups to enable use in API synthesis.

Asymmetric catalysis would provide an attractive alternative synthetic approach to overcome these shortcomings, but presents the challenge of finding a suitable chiral catalytic system having sufficient selectivity and activity for an industrially viable process. One skilled in the art might recognize catalytic asymmetric hydrogenation of an enamide precursor (**II**) as a potential process solution, but no successful routes of this type have been reported. As a tetrasubstituted olefin, enamide (**II**) has much lower activity as a hydrogenation substrate compared with standard trisubsituted enamides of formula **(III).** Asymmetric hydrogenation of the latter class of compounds, catalyzed by rhodium complexes of DuPhos and other chiral diphosphine ligands, is well known as a preferred method to make a wide range of 3-arylalanine derivatives (for representative references, see Burk et al., J. Am. Chem. Soc., 1993, 115, 10125-10138; Pye et al., J. Am. Chem. Soc., 1997, 119, 6207-6208). β-Methylphenylalanine derivatives have also been prepared using asymmetric hydrogenation of enamides of formula (**IV**) with rhodium-DuPhos and rhodium-BPE based catalysts, by the groups of Burk (J. Am. Chem. Soc., 1995, 117, 9375-76) and Turner (J. Am. Chem. Soc., 2004, 126, 4098-4099). While useful, these processes rely on the use of high catalyst loadings, high reaction pressure and lengthy reaction times.

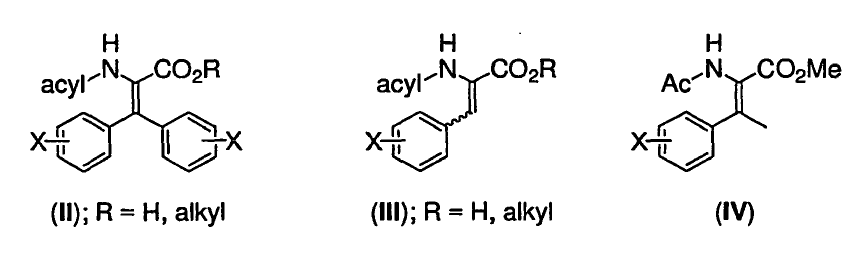

### SUMMARY OF THE INVENTION

The inventors have discovered a process for asymmetric hydrogenation of the bulky 3,3-diaryl enamides of formula II that achieves both high enantioselectivity and improved activity over processes using catalysts derived from DuPhos and BPE ligands. This due to the high activity and selectivity, there will be lower catalyst loadings required and shorter processing time, both of which can provide substantial cost savings.

The invention comprises a process for the preparation of an enantiomerically enriched 3,3-diarylalanine derivative of formula (**1**), or the opposite enantiomer thereof, which comprises asymmetric hydrogenation of the corresponding enamide of formula (**2**), in the presence of a chiral-diphosphine rhodium homogeneous hydrogenation catalyst in which the diphosphine is a PhanePhos ligand (Pye et al., J. Am. Chem. Soc., 1997, 119, 6207-6208) having the structure shown in formula (**3**). As used in this application, the language "having the structure shown in the formula" means the structure in the formula is present but additional substitutions may also be present. In (**1**) and (**2**), each of Ar¹ and Ar² is independently a C₆₋₃₀ aryl group or a C₄₋₃₀ heteroaryl group and may be unsubstituted or substituted, R¹ is H, alkyl (preferably having 1 to 10, more preferably 1-3, more preferably still 1 or 2 carbon atoms), alkoxy (preferably having 1 to 10, more preferably 1-3, more preferably still 1 or 2 carbon atoms) or aryl (preferably having 6-10, more preferably 6 carbon atoms), and R² is H or alkyl (preferably having 1 to 10, more preferably 1-3, more preferably still 1 or 2 carbon atoms). In ligand (**3**), Ar is either phenyl or a substituted phenyl group bearing one or more alkyl or alkoxy groups (preferably these alkyl or alkoxy groups have 1-10, more preferably 1-3, more preferably still 1-2, most preferably 1 carbon atom). If ligand (**3**) is substituted, the substitution is preferably on the [2.2]paracyclophane backbone. The substitution preferably is an substituted or unsubstitued aryl or aryloxy or arylalkyl or alkylaryl or of arylalkyloxy or alkylaryloxy or alkyl or alkoxy having 1 to 36 carbon atoms. For example by a trityloxymethyl group in a *para* position relative to one of the PAr₂ groups (see Hems et al., WO 2004111065). The resulting diarylalanine derivatives (**1**) are suitable for use as pharmaceutical intermediates in various applications, particularly to prepare synthetic peptides with enhanced metabolic stability. Such derivatives may also find utility in other industrial sectors that require chiral fine chemicals, such as the agrochemicals and fragrance chemicals sectors.

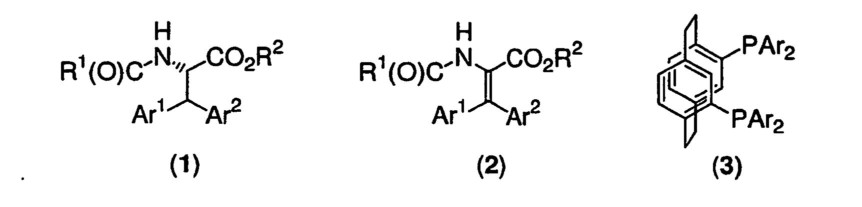

### DETAILED DESCRIPTION OF THE PREFERRED EMBODIMENTS

Asymmetric hydrogenation according to the invention comprises the steps of providing a hydrogenation substrate according to formula (**2**), and reacting with hydrogen gas in the presence of a PhanePhos ligand according to formula (**3**) complexed to rhodium. In preferred embodiments of the invention, Ar¹ = Ar² and more preferably each is phenyl or substituted phenyl, R¹ is alkyl and is more preferably methyl, R² is methyl or ethyl. Preferred ligands are PhanePhos itself (Ar = phenyl), Tol-PhanePhos (Ar = 4-methylphenyl) and Xyl-PhanePhos (Ar = 3,5-dimethylphenyl); more preferably, the ligand is PhanePhos.

For operation of the process of the invention, substrate (**2**) is dissolved in a suitable solvent, preferably an alcohol solvent selected from the group comprising methanol, ethanol, isopropanol and trifluoroethanol or mixtures thereof. More preferably, the solvent is methanol. The reaction may be run at hydrogen pressures in the range of 2-30 bar, or higher, and is preferably run at a hydrogen pressure in the range of 5-15 bar. The reaction may be run at temperatures in the range of 10-100°C, or higher, and is preferably run at a temperature in the range of 20-60°C.

Preferably, the resultant 3,3-diarylalanine derivative (**1**) is formed with an enantiomeric excess of at least 80%, more preferably of around 90% ee, or higher. If required for a specific application, the enantiomeric excess of the resultant 3,3-diarylalanine derivative (**1**) may be enhanced from about 80-90% e.e to 95% ee or higher, by the addition of a further step selected from the group consisting of crystallization, crystallization of a salt derivative or selective hydrolysis in the presence of an enantioselective biocatalyst.

The preferred catalysts demonstrated both high selectivity and activity in comparison to prior art catalysts based on alternative ligands such as bis(phosphacycles), as evidenced by the results summarized in Table 1 of Example 1, for the asymmetric hydrogenation of methyl 2-acetylamino-3,3-diphenylacrylate.

The invention is further illustrated by the following examples.

### EXAMPLE 1

The hydrogenation of methyl 2-acetylamino-3,3-diphenylacrylate, obtainable via a combination of the methods of Burk et al. (Tetrahedron Letters 1997, 38, 1309-1312) and Deng et al. (Synthesis, 2003, 337-339), was investigated according to the following standard procedure using a range of chiral diphosphine ligands. Structures of the ligands investigated are shown in Figure 1.

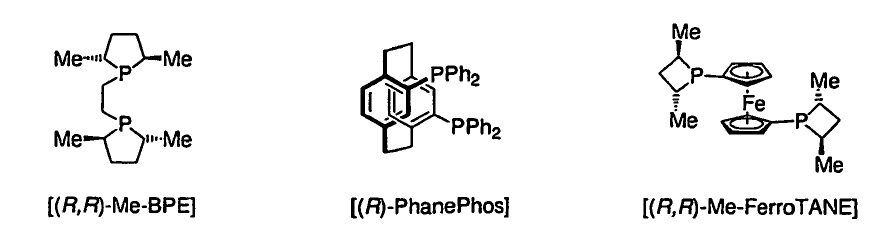

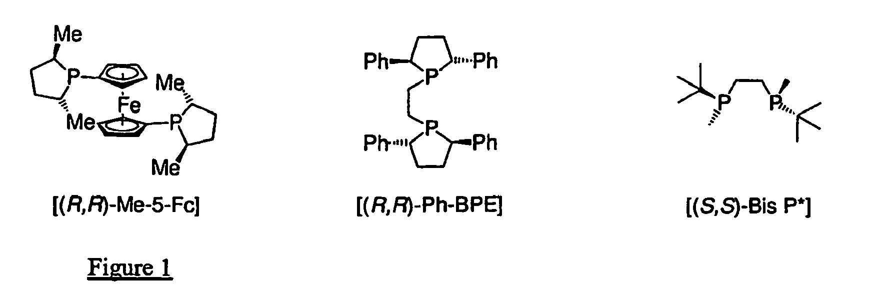

### Standard Procedure

The appropriate chiral diphosphine-rhodium hydrogenation catalyst (0.006 mmol) and methyl 2-acetylamino-3,3-diphenylacrylate (0.45 g, 1.52 mmol) were placed in a glass liner within an Argonaut Endeavor multi-well hydrogenation vessel and the vessel assembled. The vessel was pressurised to 10 bar with nitrogen and then the gas was released, this process was repeated a further three times. After the final vent, methanol (6 ml) was introduced into the bomb. The vessel was then charged and vented three times with nitrogen to 10 bar. The vessel was heated to the appropriate temperature before being charged with hydrogen to 10 bar. After stirring for 18 hours, the vessel was allowed to cool, the hydrogen pressure was released and the vessel was disassembled. The reaction mixture was concentrated under reduced pressure to afford a crude residue, which was analysed by ¹H NMR spectroscopy for conversion and SFC for enantioselectivity.

The results summarized in Table 1 demonstrate the enhanced rate and selectivity observed when catalysts based on the PhanePhos ligand system are used for this substrate type (entries 2, 4 and 9). Although comparable activities are observed with the ferrocenyl ligands shown in entries 5 and 6, such ligands are insufficiently enantioselective. The Ph-BPE ligand (entry 7) confers high enantioselectivity but activity, as determined by t_{1/2}, is unacceptably low. PhanePhos provides an optimal combination of activity and enantioselectivity.

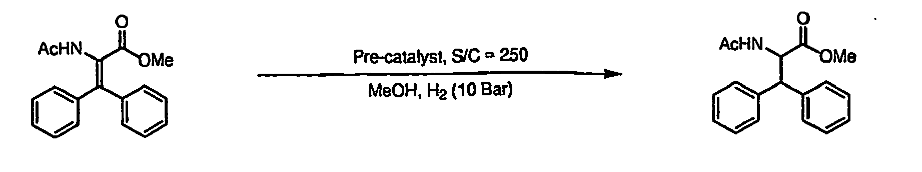

**Table 1**

| *Entry* | *Pre-catalyst* | | *Temp. (°C)* | *conv. (%)* | *e.c. (%)* |
|---|---|---|---|---|---|
| ***1*** | [(*R,R*)Me-BPE Rh COD]BF₄ | 1h 25m | 60 | 94 | 70 |
| ***2*** | [(*R*)-PhanePhos Rh COD]BF₄ | 2.5m | 60 | >95 | 88 |
| ***3*** | [(*R,R*)-Me-BPE Rh COD]BF₄ | 3h 9m | 50 | 82 | 76 |
| ***4*** | [(*R*)-PhanePhos Rh COD]BF₄ | 3m | 50 | >95 | 88 |
| ***5*** | [(*R,R*)-Me-FerroTANE Rh COD]BF₄ | 13m | 50 | 72 | 72 |
| ***6*** | -[(*R,R*)-Me-5-Fc Rh COD]BF₄ | 2m | 50 | >95 | 75 |
| ***7*** | [(*R,R*)-Ph-BPE Rh COD]BF₄ | 3h 23m | 50 | 96 | 94 |
| ***8*** | [(*S,S*)-Bis P Rh COD]BF₄ | 5h 17m | 50 | 73 | 51 |
| ***9*** | [(*R*)-PhanePhos Rh COD]BF₄ | nd | 20 | >95 | 89 |
| ***10*** | [(*R,R*)-Me-BPE Rh COD]BF₄ | nd | 20 | 95 | 78 |

| | | | | | |
|---|---|---|---|---|---|
| # Conversion assessed by analysis of 1H nmr spectrum of the crude material. * Half life determined from hydrogen consumption from normalised plots. | | | | | |

## Claims

1. A process for the preparation of an enantiomerically enriched 3,3-diarylalanine derivative of formula (**1**), or the opposite enantiomer thereof, wherein each of Ar¹ and Ar² is independently a C₆₋₃₀ aryl group or a C₄₋₃₀ heteroaryl group and may be unsubstituted or substituted, R¹ is H, alkyl, alkoxy or aryl, and R² is H or alkyl, which comprises asymmetric hydrogenation of the corresponding enamide of formula (**2**), in the presence of a chiral-diphosphine rhodium homogeneous hydrogenation catalyst in which the diphosphine is a ligand having the structure shown in formula (**3**), wherein Ar is either phenyl or a substituted phenyl group bearing one or more alkyl or alkoxy groups.

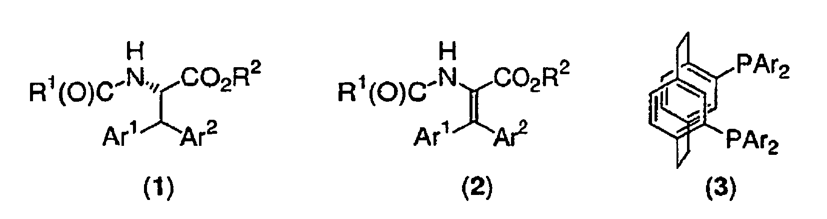

2. The process of claim 1, wherein Ar¹ = Ar².

3. The process of claim 2, wherein each of Ar¹ and Ar² is phenyl or substituted phenyl.

4. The process of claim 1, wherein R¹ is alkyl.

5. The process of claim 4, wherein R¹ is methyl.

6. The process of claim 1, wherein R² is alkyl.

7. The process of claim 6, wherein R² is methyl or ethyl.

8. The process of claim 1, wherein Ar in the ligand (**3**) is selected from the group consisting of phenyl, 4-methylphenyl and 3,5-dimethylphenyl.

9. The process of claim 8, wherein Ar in the ligand (**3**) is phenyl.

10. The process of claim 1, wherein the product (**1**) is formed with an enantiomeric excess of at least 80%.

11. The process of claim 1, wherein the product (**1**) is formed with an enantiomeric excess of at least 90%.

12. The process of claim 1 wherein there is no additional substitution on the ligand (**3**).

13. The process of claim 1 wherein the [2.2]paracyclophane backbone of ligand (**3**) is substituted.

14. A process according to claim 1, which comprises a further step selected from the group consisting of crystallization, crystallization of a salt derivative or selective hydrolysis in the presence of an enantioselective biocatalyst, in order to increase enantiomeric excess of the product (**1**) or a derivative thereof.

## Patentansprüche

1. Verfahren zur Herstellung eines enantiomerenangereicherten 3,3-Diarylalanin-Derivats der Formel (1) oder des entgegengesetzten Enantiomers davon, wobei Ar¹ und Ar² jeweils unabhängig voneinander eine C₆₋₃₀-Arylgruppe oder eine C₄₋₃₀-Heteroarylgruppe sind und substituiert oder nicht substituiert sein können, R¹ H, Alkyl, Alkoxy oder Aryl ist und R² H oder Alkyl ist, wobei das Verfahren den Schritt der asymmetrischen Hydrierung des entsprechenden Enamids der Formel (2) in Gegenwart eines homogenen Hydrierkatalysators in Form von chiralem Diphosphinrhodium umfasst, bei dem das Diphosphin ein Ligand mit der in Formel (3) dargestellten Struktur ist, wobei Ar entweder Phenyl oder eine substituierte Phenylgruppe ist, die eine oder mehr Alkyl- oder Alkoxygruppen trägt.

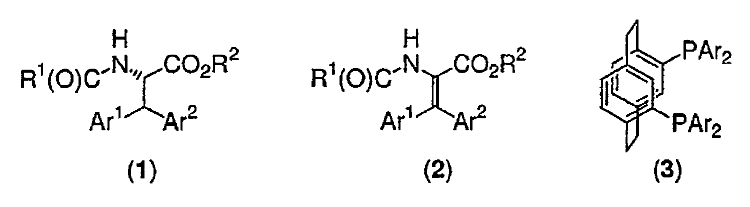

2. Verfahren nach Anspruch 1, wobei Ar¹ = Ar².

3. Verfahren nach Anspruch 2, wobei Ar¹ und Ar² jeweils Phenyl oder substituiertes Phenyl sind.

4. Verfahren nach Anspruch 1, wobei R¹ Alkyl ist.

5. Verfahren nach Anspruch 4, wobei R¹ Methyl ist.

6. Verfahren nach Anspruch 1, wobei R² Alkyl ist.

7. Verfahren nach Anspruch 6, wobei R² Methyl oder Ethyl ist.

8. Verfahren nach Anspruch 1, wobei Ar in dem Liganden (3) aus der aus Phenyl, 4-Methylphenyl und 3,5-Dimethylphenyl bestehenden Gruppe ausgewählt ist.

9. Verfahren nach Anspruch 8, wobei Ar in dem Liganden (3) Phenyl ist.

10. Verfahren nach Anspruch 1, wobei das Produkt (1) mit einem Enantiomerenüberschuss von mindestens 80% gebildet wird.

11. Verfahren nach Anspruch 1, wobei das Produkt (1) mit einem Enantiomerenüberschuss von mindestens 90% gebildet wird.

12. Verfahren nach Anspruch 1, wobei es an dem Liganden (3) keine weitere Substitution gibt.

13. Verfahren nach Anspruch 1, wobei die [2.2]Paracyclophan-Hauptkette des Liganden (3) substituiert ist.

14. Verfahren nach Anspruch 1, das einen weiteren aus der aus Kristallisation, Kristallisation eines Salzderivats oder selektive Hydrolyse in Gegenwart eines enantioselektiven Biokatalysators bestehenden Gruppe ausgewählten Schritt umfasst, um den Enantiomerenüberschuss des Produkts (1) oder eines Derivats davon zu erhöhen.

## Revendications

1. Procédé de préparation d'un dérivé de 3,3-diaryl-alanine enrichi en l'énantiomère de formule (1) ou en l'énantiomère opposé, dans laquelle formule Ar¹ et Ar² représentent chacun, indépendamment, un groupe aryle en C₆₋₃₀ ou hétéroaryle et C₄₋₃₀, avec ou sans substituant(s), R¹ représente un atome d'hydrogène ou un groupe alkyle, alcoxy ou aryle, et R² représente un atome d'hydrogène ou un groupe alkyle, lequel procédé comporte une hydrogénation asymétrique de l'énamide correspondant de formule (2), réalisée en présence d'un catalyseur d'hydrogénation en phase homogène qui est un composé chiral de diphosphine et de rhodium dans lequel la diphosphine est un ligand de structure représentée par la formule (3) dans laquelle Ar représente soit un groupe phényle, soit un groupe phényle portant en tant que substituant(s) un ou plusieurs groupes alkyle ou alcoxy.

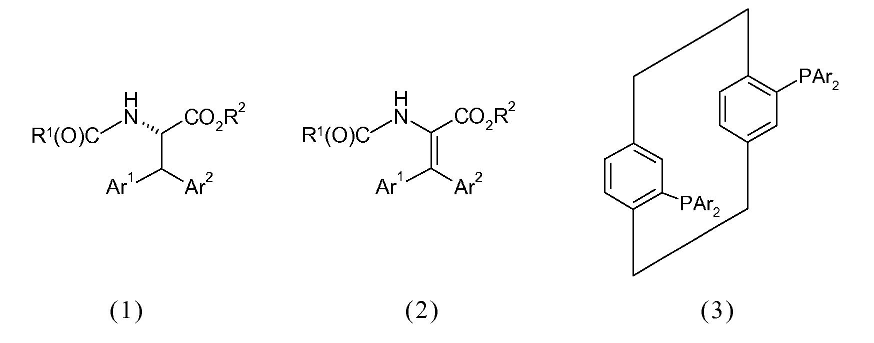

2. Procédé conforme à la revendication 1, dans lequel les groupes représentés par Ar¹ et Ar² sont identiques.

3. Procédé conforme à la revendication 2, dans lequel Ar¹ et Ar² représentent chacun un groupe phényle ou un groupe phényle à substituant(s).

4. Procédé conforme à la revendication 1, dans lequel R¹ représente un groupe alkyle.

5. Procédé conforme à la revendication 4, dans lequel R¹ représente un groupe méthyle.

6. Procédé conforme à la revendication 1, dans lequel R² représente un groupe alkyle.

7. Procédé conforme à la revendication 6, dans lequel R² représente un groupe méthyle ou éthyle.

8. Procédé conforme à la revendication 1, dans lequel, dans le ligand de formule (3), Ar représente un groupe choisi parmi les groupes phényle, 4-méthyl-phényle et 3,5-diméthyl-phényle.

9. Procédé conforme à la revendication 8, dans lequel, dans le ligand de formule (3), Ar représente un groupe phényle.

10. Procédé conforme à la revendication 1, dans lequel le produit de formule (1) se forme en un excès énantiomérique d'au moins 80 %.

11. Procédé conforme à la revendication 1, dans lequel le produit de formule (1) se forme en un excès énantiomérique d'au moins 90 %.

12. Procédé conforme à la revendication 1, dans lequel, dans le ligand de formule (3), il n'y a aucun substituant supplémentaire.

13. Procédé conforme à la revendication 1, dans lequel, dans le ligand de formule (3), il y a un ou des substituant(s) sur le squelette [2.2]paracyclophane.

14. Procédé conforme à la revendication 1, qui comporte une étape de plus, choisie parmi la cristallisation du produit, la cristallisation d'un dérivé de type sel et l'hydrolyse sélective du produit en présence d'un bio-catalyseur énantiosélectif, et réalisée afin d'obtenir le produit de formule (1) ou un dérivé de celui-ci en un excès énantiomérique encore plus élevé.
